# EUROPEAN PATENT SPECIFICATION

(11) **EP 3 240 526 B1**
(45) Date of publication and mention of the grant of the patent: **08.07.2020**
(21) Application number: 15805228.2
(22) Date of filing: 08.12.2015
(51) Int. Cl.: A61K 8/49, A61K 8/67, A61Q 19/02

(54) **A SKIN LIGHTENING COMPOSITION COMPRISING NIACINAMIDE AND ILOMASTAT**
HAUTAUFHELLUNGSZUSAMMENSETZUNG ENTHALTEND NIACINAMID UND ILOMASTAT
COMPOSITION POUR ÉCLAIRCIR LA PEAU CONTENANT DE LA NIACINAMIDE ET DE L'ILOMASTAT

(30) Priority: 30.12.2014 EP 14200619
(43) Date of publication of application: 08.11.2017
(73) Proprietor: Unilever N.V., 3013 AL Rotterdam (NL); Unilever PLC, a company registered in England and Wales under company no. 41424, Greater London EC4Y 0DY (GB)
(72) Inventor: DUTTA, Maitreyee, Whitefield Bangalore 560 066 (IN); KALE, Vaidehi,Subhash, Pune Maharashtra 411016 (IN); NAIR, Nirmala, Santosh, Whitefield Bangalore 560 066 (IN)
(74) Representative: James, Helen Sarah
(86) International application number: PCT/EP2015/078975
(87) International publication number: WO 2016/107720

(56) References cited:
- EP-A1- 2 412 701
- EP-A1- 2 522 331
- WO-A2-02/19982
- US-A1- 2010 158 842
- LIN X ET AL: "GM6001, a broad spectrum metalloprotease inhibitor, has multiple efficacies against skin aging", THE JOURNAL OF INVESTIGATIVE DERMATOLOGY, NATURE PUBLISHING GROUP, GB, 1 April 2010 (2010-04-01), XP009141234, ISSN: 0022-202X

## Description

### Field of the invention

The present invention is in the field of personal care compositions; in particular skin lightening compositions.

### Background of the invention

Most people are concerned with the degree of pigmentation of their skin. For example, people with age spots or freckles may wish such pigmented spots to be less pronounced. Others may wish to reduce the skin darkening caused by exposure to sunlight or to lighten their natural skin colour. To meet this need, many attempts have been made to develop products that reduce the pigment production in the melanocytes. However, the substances identified thus far tend to have either low efficacy or undesirable side effects, such as, for example, toxicity or skin irritation. Therefore, there is a continuing need for new cosmetic skin lightening agents, with improved overall effectiveness.

Conventional skin lightening compositions are based on use of skin lightening agents that are believed to control dispersion of melanin or inhibit tyrosinase. These skin-lightening agents include niacinamide, carboxylic acids like azelaic acid and kojic acid, plant extracts and hydroquinone etc. Niacinamide, which is a Vitamin B3 compound, is one such widely used skin lightening agent in compositions for topical application.

DE 10133196 (Beiersdorf) discloses topical compositions containing nicotinic acid or its precursors, derivatives or metabolites, used to improve skin condition and (1) to treat or prevent dry skin, to enhance the barrier function of the skin, for treatment, care and prophylaxis of sensitive skin and/or for treatment and prophylaxis of symptoms of a negative change in the physiological homeostasis of normal skin; (2) to treat or prevent inflammatory skin conditions, atopic eczema, polymorphic photodermatitis, psoriasis, vitiligo and sensitive, itching or irritated skin; (3) to treat or prevent abnormal lipid peroxidation; (4) to treat or prevent abnormal ceramide, lipid and energy metabolism of the skin, abnormal trans epidermal water loss, reduced skin hydration and moisture content and altered Natural Moisturizing Factor content; (5) to treat or prevent reduced cell-cell communication, deficient intracellular DNA synthesis, DNA damage, reduced endogenous DNA repair, activation of metalloproteinases and/or other proteases or inhibition of corresponding DNA repair mechanisms and abnormal post-translational modification of connective tissue components; (6) to treat or prevent abnormal skin hyaluronic acid and glucosamino glycan content; and (7) to treat or prevent dandruff of the hair and scalp and skin aging.

EP 2 522 331 A1 discloses a synergistic skin lightening composition comprising niacinamide and resveratrol. Galardin is not disclosed.

"GM6001, a broad spectrum metalloprotease inhibitor, has multiple efficacies against skin aging", Lin X et al., The Journal of Investigative Dermatology, Nature Publishing Group, GB (2010-04-01) discloses that GM6001 (galardin, ilomastat) exhibits a whitening effect. Niacinamide is not disclosed.

EP 2 412 701 A1 discloses indoyl amide derivatives having a suppression effect on melanin production and skin whitening compositions comprising indoyl amide derivatives alone or in combination with other whitening agents. Galardin and niacinamide are both not disclosed.

US 2010/158842 A1 discloses a cosmetic skin whitening composition comprising Tasselin A (oxindole compound) as melanin inhibitor and as anti-tyrosinase enzyme inhibitor. Galardin and niacinamide are both not disclosed.

It is believed that niacinamide reduces the effective concentration of cyclic adenylic acid (cyclic 3'-5'-AMP) which is thought to act as the second messenger mediating the action of various hormones including the melanocyte stimulating hormone involved in the dispersion of melanin. Cyclic 3' - 5'-AMP phosphodiesterase is an enzyme which degrades cyclic 3'-5'-AMP to adenosine-5'-monophosphate. The activity of this enzyme has been shown to be stimulated by niacinamide. Thus niacinamide is believed to reduce the effective concentration of cyclic 3'-5'-AMP and hence the dispersion of melanin granules. Additionally, niacinamide has been shown to reduce melanosome transfer from melanocytes to keratinocytes. However, niacinamide is known to have little or no effect directly on the melanin content in melanocytes.

A composition that combines the cyclic adenylic acid reduction and the reduction of melanin content in melanocytes remains to be desired.

It has been found that that niacinamide in combination with MMP inhibitor galardin synergistically reduces melanin content in melanocytes. The invention thus relates to a composition comprising a synergistic combination of niacinamide and galardin for use in skin lightening. Thus the composition, when applied topically over an appropriate length of time in-vivo, may be used to lighten the skin, or to reduce age spots or freckles.

### Summary of the invention

Accordingly, in a first aspect, the present invention provides a skin lightening composition comprising an effective amount of niacinamide; and an effective amount of galardin.

In a second aspect, the present invention provides use of the composition according to the invention for skin lightening.

In a third aspect, the invention provides a method of lightening the skin of a human, the method comprising the step of applying the composition according to the invention onto the skin.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

### Detailed description of the invention

In a first aspect of the invention, a skin lightening composition is provided, the composition comprising niacinamide and galardin.

### Niacinamide

Niacinamide also known as nicotinamide and as pyridine-3-carboxamide is the active, water soluble form of vitamin B3. It is essential to the coenzymes NADH and NADPH and therefore for over 200 enzymatic reactions in the body including ATP formation.

The composition of the present invention comprises an effective amount of niacinamide, typically in a concentration of 0.001 to 10%, preferably at least 0.01%, more preferably at least 0.1%, still more preferably at least 1%, even more preferably at least 2%, yet more preferably at least 3%, or even at least 4% by weight of the composition; but preferably not more than 9%, more preferably not more than 8%, still more preferably not more than 7%, yet more preferably not more than 6%, or even not more than 5% by weight of the composition.

### Galardin

Galardin also known as GM6001 or ilomastat is a broad-spectrum matrix metalloproteinase inhibitor having the following structure:

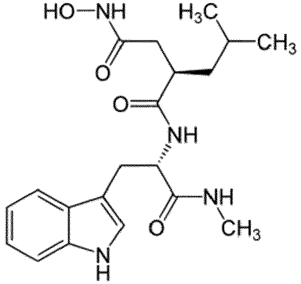

Galardin is a member of the hydroxamic acid class of reversible matrix metalloproteinase inhibitors. The anionic state of the hydroxamic acid group forms a bidentate complex with the active site zinc.

Matrix metalloproteinases (MMPs) are known for their role in matrix remodeling and are secreted from a variety of cells, including skin cells such as melanocytes, keratinocytes and fibroblast. Galardin is a broad-spectrum synthetic matrix metalloproteinase (MMP) inhibitor, with potent activity against MMP-1, 2, 3, 7, 8, 9, 12, 14, 26. First reported in the 1990s, it has a collagen-like backbone to facilitate binding to the active site of MMPs and a hydroxamate structure (R-CO-NH-OH, where R is an organic residue) which chelates the zinc ion located in the catalytic domain of MMPs. It has been reported to modulate MMPs by preventing the conversion of pro-MMPs into their active form.

Considering that melanin is a group of pigments formed by the oxidation of the amino acid tyrosine, followed by polymerization, it is surprising that galardin also plays a role in the reduction of melanin content in melanocytes together with niacinamide. Additionally Galardin also does not appear to have an effect on the inhibition of tyrosinase, which is an oxidase for tyrosine and is a copper containing protein, but not a metalloproteinase, as is demonstrated in the examples herein below.

The present invention highlights the role of galardin in synergistically modulating melanin content in primary human melanocytes by combining with niacinamide.

The composition of the present invention comprises an effective amount of galardin, typically in a concentration of 0.00001 to 10%, preferably at least 0.0001 %, more preferably at least 0.001%, still more preferably at least 0.001%, even more preferably at least 0.1%, yet more preferably at least 0.5%, or even at least 1% by weight of the composition; but preferably not more than 8%, more preferably not more than 5%, still more preferably not more than 4%, even more preferably not more than 3%,yet more preferably not more than 2% or even not more than 1.5% by weight of the composition.

### Other Ingredients

The composition of the present invention may further comprise a cosmetically acceptable vehicle, which may act as diluents, dispersants and/or carriers for the skin lightening agents used in the composition, so as to facilitate their distribution when the composition is applied to the skin. The cosmetically acceptable vehicle suitable for use in the present invention may be aqueous, anhydrous or an emulsion; aqueous or an emulsion, especially water-in-oil or oil-in-water emulsion being most preferred. Water when present typically makes up the balance of the composition. Preferably water is present in a concentration of 5 to 99%, more preferably from 20 to 80%, still more preferably from 40 and 80% by weight of the composition.

Besides water, organic solvents may also serve as carriers within compositions of the present invention.

Emollients may also be used as cosmetically acceptable carrriers in the composition of the present invention. Emollients are generally in the form of silicone oils and synthetic esters. Silicone oils may be volatile and non-volatile. Volatile silicone oils are preferably chosen from cyclic or linear polydimethylsiloxanes containing from 3 to 9, preferably from 4 to 5, silicon atoms. Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes.

Ester emollients that may be used are:
a) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
b) Ether-esters such as fatty acid esters of ethoxylated fatty alcohols.
c) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
d) Wax esters such as beeswax, spermaceti, myristyl myristate, stearyl stearate and arachidyl behenate.
e) Sterols esters, of which cholesterol fatty acid esters are examples.

Emollients may be present in the composition anywhere from 0.1 to 50%, preferably from 1 to 20% by weight of the composition.

Fatty acids having from 10 to 30 carbon atoms may also be included as cosmetically acceptable carriers in the composition of this invention. Illustrative examples of such fatty acids are pelargonic, lauric, myristic, palmitic, stearic, isostearic, hydroxystearic, oleic, linoleic, ricinoleic, arachidic, behenic, erucic acids and mixtures thereof.

Humectants of the polyhydric alcohol type may also be employed as cosmetically acceptable carriers in the composition of this invention. The humectant aids in increasing the effectiveness of the emollient, reduces scaling, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably propylene glycol or sodium hyaluronate. The concentration of humectant in the composition may range anywhere from 0.5 to 30%, preferably between 1 and 15% by weight of the composition.

Thickeners may also be utilized as part of the cosmetically acceptable carrier of compositions according to the present invention. Typical thickeners include crosslinked acrylates (e.g. Carbopol 982), hydrophobically-modfied acrylates (e.g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Concentration of the thickener in the composition may range from 0.0001 to 5%, usually from 0.001 to 1%, optimally from 0.01 to 0.5% by weight.

Collectively the water, solvents, silicones, esters, fatty acids, humectants and/or thickeners will constitute the cosmetically acceptable carrier in amounts from 1 to 99.9%, preferably from 80 to 99% by weight of the composition.

Surfactants may also be present in the composition of the present invention. Total concentration of the surfactant will range from 0.1 to 40%, preferably from 1 to 20%, optimally from 1 to 5% by weight of the composition. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C₈-C₂₀ acyl isethionates, acyl glutamates, C₈-C₂₀ alkyl ether phosphates and combinations thereof.

Sunscreens include those materials commonly employed to block ultraviolet light. Illustrative compounds are the derivatives of PABA, cinnamate and salicylate. For example, avobenzophenone (Parsol 1789®) octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed in the compositions can vary depending upon the degree of protection desired from the sun's UV radiation. Additives that reflect or scatter the sun rays may also be employed. These additives include oxides like zinc oxide and titanium dioxide.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, polymers, astringents, fragrance, opacifying agents, conditioners, exfoliating agents, pH adjusters, preservatives, natural extracts, essential oils, skin sensates, skin soothing agents, and skin healing agents.

When making the composition of the present invention, the desired ingredients are mixed in no particular order and usually at temperatures from about 70 to about 80°C and under atmospheric pressure.

The packaging for the composition of this invention can be a patch, bottle, tube, roll-ball applicator, propellant driven aerosol device, squeeze container or lidded jar.

In a second aspect, the invention relates to use of the composition according to the invention for skin lightening.

In a third aspect, the invention relates to a method of lightening the skin of a human, the method comprising the step of applying the composition according to the invention onto the skin.

The invention will now be illustrated by means of the following non-limiting examples.

### Examples

### Example 1: In-vitro studies on the effect of niacinamide and galardin on melanin inhibition

### Materials

(i) Primary human neonatal foreskin melanocytes, Medium 254 and Human Melanocyte Growth Supplement-2 (Life Technologies)
(ii) Galardin(R)-N4-Hydroxy-N1-[(S)-2-(1H-indol-3-yl)-1-methylcarbamoyl-ethyl]-2-isobutyl-succinamide (Sigma-Aldrich)
(iii) Nicotinamide (Sigma Aldrich)
(iv) Hexylresorcinol (Sigma Aldrich)
(v) Calcein-AM - Calcein acetoxymethyl ester (Sigma Aldrich)
(vi) DMSO - Dimethyl sulfoxide (Sigma Aldrich)
(vii) Sodium hydroxide (Merck Specialities Pvt. Ltd.)
(viii) HEPES-(4-(2-Hydroxyethyl)piperazine-1-ethanesulfonic acid, N-(2-Hydroxyethyl)piperazine-N'-(2-ethanesulfonic acid) (Sigma Aldrich)
(ix) NaCl - Sodium chloride (Fisher Scientific)
(x) NP40 - Nonidet P 40 (USB Corporation)
(xi) PMSF - Phenylmethanesulfonyl fluoride (Sigma-Aldrich)
(xii) Protease inhibitor cocktail (Sigma-Aldrich)
(xiii) L-Tyrosine (Sigma-Aldrich)

### Methods

### (i) Cell cultures:

Neonatal foreskin primary human melanocytes were procured from Life Technologies, USA. Cells were grown in Medium 254 supplemented with human melanocyte growth supplement-2 and maintained at 37°C in a humidified incubator with 5% CO₂ atmosphere. The maintenance and sub culturing of cells were carried out as per the manufacturer's instructions. Cells between passages 3-6 were used for experimentation.

### (ii) Active addition:

Cells were seeded at a density of 5X104 /well in a 24 well plate in Melanocyte Growth Media (MGM) and incubated for 24 hrs at 37°C/5% CO₂. Actives were added to cells along with relevant controls. 72hrs post active treatment, cell viability was determined using the Calcein method followed by measurement of cellular melanin content (described in below sections).

### (iii) Cell Viability assay

Briefly, spent media was removed and cells washed once with 0.2ml of 1x PBS-Ca-Mg solution. Fresh 1 µM calcein-AM in PBS buffer was added to each well. Plates were incubated for 30 min. at 37°C in a CO2 incubator. Calcein fluorescence was then measured (excitation at 490 nm and emission at 520 nm) in TECAN M1000 plate reader.

### (iv) Melanin content assay:

After Calcein measurements, cultures were rinsed with PBS (1x) and lysed for 1 hour at 60°C in a shaker incubator using NaOH/DMSO mix. Melanin content was measured in a Tecan plate reader (405nm filter), corrected for cell numbers and represented as %inhibition.

### (v) In vitro Human Tyrosinase activity assay:

Primary human melanocytic lysate was used as a source of Tyrosinase enzyme. Human melanocytic lysate and test actives (at different concentrations) were incubated together for 15mins, prior to addition of Tyrosine/DOPA substrate mix. Assay plate was then incubated at 37°C for -14-16 hrs. Optical Density was measured at 405 nm and % Inhibition calculated.

### Results

**Table I**

| **Treatments** | **Average % inhibition in cellular melanin** | **SE** |
|---|---|---|
| DMSO 0.1% | 0 | 0 |
| 1uM Galardin | 6 | 3 |
| 10uM Galardin | 17 | 4 |
| 1mM Niacinamide | -7 | 3 |
| 10mM Niacinamide | 16 | 4 |
| DMSO 0.2% | 0 | 0 |
| 1mM Niacinamide + 1uM Galardin | 13 | 4 |
| 1mM Niacinamide+ 10uM Galardin | 25 | 4 |
| 10mM Niacinamide + 1uM Galardin | 31 | 4 |
| 10mM Niacinamide + 10uM Galardin | 37 | 4 |

**Table II**

| **Treatments** | **Average % inhibition in Tyrosinase enzyme activity** | **SE** |
|---|---|---|
| No enzyme control | 100 | 0 |
| 50µM Galardin | -20 | 11 |
| 10µM Galardin | -2 | 14 |
| 5µM Galardin | -3 | 15 |
| 1µM Galardin | -1 | 10 |
| 50mM Niacinamide | -20 | 12 |
| 10mM Niacinamide | -8 | 3 |
| 1mM Niacinamide | 4 | 6 |
| 0.1mM Niacinamide | 2 | 6 |
| 0.01mM Niacinamide | 2 | 11 |

### Conclusion

The above table (table I) highlights the synergistic reduction of melanin content in melanocytes obtained by combining galardin with niacinamide. The data in table I shows that galardin by itself gave either no reduction (at 1µM) or modest reduction (at 10 µM) of melanin content in primary human melanocytes. However, when galardin and niacinamide were combined together in different ratios, synergistic reduction in melanin content was obtained at specific tested concentrations of each active.

Table II further illustrates that such reduction in melanin content was not through direct modulation of tyrosinase enzyme activity. Similar results were seen with niacinamide also.

## Claims

1. A skin lightening composition comprising:
a) An effective amount of niacinamide; and
b) An effective amount of galardin.

2. A skin lightening composition according to claim 1 wherein the composition comprises 0.00001 to 10% by weight of galardin.

3. A skin lightening composition according to claim 1 or claim 2 wherein the composition comprises 0.001 to 10% by weight of niacinamide.

4. A skin lightening composition according to claims 1 to 3 further comprising a skin lightening agent.

5. A skin lightening composition according to claims 1 to 4 in the form of a topical composition.

6. Use of the composition according to claims 1 to 5 for skin lightening.

7. A method of lightening the skin of a human, the method comprising the step of applying the composition according to claims 1 to 5 onto the skin.

## Patentansprüche

1. Hautaufhellungszusammensetzung, umfassend:
a) eine wirksame Menge Niacinamid; und
b) eine wirksame Menge Galardin.

2. Hautaufhellungszusammensetzung nach Anspruch 1, wobei die Zusammensetzung 0,00001 bis 10 Gewichts-% Galardin umfasst.

3. Hautaufhellungszusammensetzung nach Anspruch 1 oder Anspruch 2, wobei die Zusammensetzung 0,001 bis 10 Gewichts-% Niacinamid umfasst.

4. Hautaufhellungszusammensetzung nach den Ansprüchen 1 bis 3, die ferner ein Hautaufhellungsmittel umfasst.

5. Hautaufhellungszusammensetzung nach den Ansprüchen 1 bis 4 in Form einer topischen Zusammensetzung.

6. Verwendung der Zusammensetzung nach den Ansprüchen 1 bis 5 zum Aufhellen der Haut.

7. Verfahren zum Aufhellen der Haut eines Menschen, wobei das Verfahren den Schritt des Auftragens der Zusammensetzung nach den Ansprüchen 1 bis 5 auf die Haut umfasst.

## Revendications

1. Composition éclaircissant la peau comprenant :
a) une quantité efficace de niacinamide ; et
b) une quantité efficace de galardine.

2. Composition éclaircissant la peau selon la revendication 1, dans laquelle la composition comprend de 0,00001 à 10 % en masse de galardine.

3. Composition éclaircissant la peau selon la revendication 1 ou revendication 2, dans laquelle la composition comprend de 0,001 à 10 % en masse de niacinamide.

4. Composition éclaircissant la peau selon les revendications 1 à 3 comprenant de plus un agent éclaircissant la peau.

5. Composition éclaircissant la peau selon les revendications 1 à 4 dans la forme d'une composition topique.

6. Utilisation de la composition selon les revendications 1 à 5 pour l'éclaircissement de la peau.

7. Procédé d'éclaircissement de la peau d'un humain, le procédé comprenant l'étape d'application de la composition selon les revendications 1 à 5 sur la peau.
